# DEMANDE DE BREVET EUROPEEN

(11) **EP 1 535 608 A1**
(43) Date de publication de la demande: **01.06.2005**
(21) Numéro de dépôt: 04292532.1
(22) Date de dépôt: 26.10.2004
(51) Int. Cl.: A61K 7/48, A61K 7/06, A61K 7/00, A45D 31/00, A41G 3/00, A41G 5/02

(54) **Composition cosmétique apte à former une matrice polymérique comportant des reliefs creux ou des excroissances**

(30) Priorité: 28.11.2003 FR 0350935
(71) Demandeur: L'OREAL, 75008 Paris (FR)
(72) Inventeur: Vic, Gabin, 60280 Venette (FR); Livoreil, Aude, 75006 Paris (FR); Samain, Henri, 91570 Bievres (FR); Heinzelmann, Harry, 2000 Neuchatel (CH); Pugin, Rapha l, 2012 Auvernier (CH); Jeney, Sylvia, 1025 St Sulpice (CH)
(74) Mandataire: Bourdeau, Françoise

(57) **Abrégé**

L'invention concerne une composition cosmétique comprenant, dans un milieu physiologiquement acceptable, n polymères (P₁, P₂,.....Pₙ) avec 10≥n≥2, solubilisés dans un solvant commun (S_{c}), les polymères (P₁, P₂,.....Pₙ) et le solvant commun (S_{c}), lequel est liquide à la température ambiante et à la pression atmosphérique, étant choisis, pour former des domaines distincts, constitués, chacun, d'un seul polymère, choisi parmi P₁, P₂,..... ou Pₙ, après dépôt de la composition cosmétique sur un substrat en matière kératinique humaine et élimination subséquente du solvant commun (S_{c}).

## Description

L'invention concerne une composition cosmétique apte à former une matrice polymérique comportant des reliefs creux ou des excroissances. Elle vise également un kit comprenant une telle composition, un substrat recouvert par cette composition et un procédé cosmétique comprenant l'application de cette composition.

Le terme *"matière kératinique"* englobe la peau, les ongles et les fibres kératiniques. On entend par "fibres kératiniques", les cheveux, les cils, les sourcils, les moustaches, les poils et notamment les cheveux. La présente invention vise, en particulier, les fibres kératiniques.

Par le terme *"substitut de matière kératinique",* on entend les accessoires de maquillage ou de soin en matière naturelle ou synthétique, servant d'accessoire de remplacement (support) à la matière kératinique humaine. Parmi ceux-ci, on peut citer les faux ongles, faux cils, postiches, mèches, perruques ou encore des pastilles ou patchs adhérents sur la peau ou les lèvres (du type mouches).

Avec l'évolution de la mode, les consommateurs, plus exigeants, recherchent des produits cosmétiques pour la peau ou les cheveux procurant des effets cosmétiques, notamment de maquillage, originaux ou particuliers. En particulier, un besoin existe de disposer de produits cosmétiques, dont l'application sur un support comme la peau ou fibres kératiniques d'êtres humains donne lieu à un effet de maquillage, différent de ceux des films continus et homogènes actuellement obtenus avec les produits disponibles sur le marché.

En outre, on recherche de tels effets cosmétiques, notamment de maquillage, qui forment un relief original, conférant du volume ou du gonflant aux matières kératiniques, notamment aux fibres kératiniques. En outre, le relief doit présenter une bonne adhésion sur le support maquillé et une bonne tenue, notamment aux frottements et aux chocs.

Sur les cheveux, on recherche la formation de reliefs structurés apportant, en outre, de bonnes propriétés cosmétiques. Les reliefs peuvent apporter des effets de rugosité, procurant du coiffage, une texture ou un nouveau toucher, des effets optiques, comme une couleur, un reflet ou une brillance anti-réflexion ou des effets d'hydrophobisation donnant lieu à un séchage rapide.

Pour certaines applications, le revêtement recherché peut, par exemple, présenter un effet granité ou de "crépi" sur le support où il est appliqué. On peut aussi créer, sur le substrat en matière kératinique ou substitut, des effets attractifs du point de vue esthétique et/ou permettant de camoufler des imperfections, telles que, par exemple, des rides, ridules, boutons, rougeurs ou couperose. En particulier, on peut créer un effet de contraste pour dissimuler un défaut ou détourner le regard d'une imperfection ou encore pour modifier un état de surface.

On peut également chercher à former un revêtement produisant une image optiquement variable, qui peut encore jouer le rôle d'une mouche. On peut encore chercher la création de couleurs par un seul phénomène d'interférences, éventuellement sans utiliser nécessairement de colorants solubles dans le milieu cosmétiquement acceptable ou de pigments spécifiques, c'est-à-dire sans poser les difficultés de compatibilité, toxicologie, réglementation, rencontrées lors de la mise au point de nouvelles compositions comportant des colorants ou pigments. Ainsi, le revêtement utilisé peut, sans pigment ou colorant, donner naissance à la formation de reflet ou de couleur.

Pour d'autres applications, on recherche des microreliefs pour constituer un réseau de diffraction. Dans certains cas, on recherche une image optiquement variable pour produire un effet goniochromatique ou encore un effet holographique.

Dans d'autres cas encore, on recherche la modification des propriétés de mouillage ou de démouillage des matières kératiniques humaines ou substituts ou, aussi, la modification de leur surface pour faire varier la tenue d'un produit de soin ou de maquillage.

Le problème posé par l'invention est de fournir des compositions cosmétiques répondant mieux que celles de l'art antérieur aux différentes exigences exprimées ci-dessus.

Pour résoudre ce problème, l'invention propose une composition cosmétique comprenant, dans un milieu physiologiquement acceptable, n polymères (P₁, P₂,......Pₙ) avec 10≥n≥2, solubilisés dans un solvant commun (S_{c}), les polymères (P₁, P₂,......Pₙ) et le solvant commun (S_{c}), lequel est liquide à la température ambiante et à la pression atmosphérique, étant choisis, pour former des domaines distincts, constitués, chacun, d'un seul polymère, choisi parmi P₁, P₂,...... ou Pₙ, après dépôt de la composition cosmétique sur un substrat en matière kératinique humaine et élimination subséquente du solvant commun (S_{c}).

Un autre objet de l'invention concerne un kit comprenant (i) une première composition cosmétique comprenant, dans un milieu physiologiquement acceptable, n polymères (P₁, P₂,......Pₙ) avec 10≥n≥2, solubilisés dans un solvant commun (S_{c}), les polymères (P₁, P₂,......Pₙ) et le solvant commun (S_{c}), lequel est liquide à la température ambiante et à la pression atmosphérique, étant choisis, pour former des domaines distincts, constitués, chacun, d'un seul polymère, choisi parmi P₁, P₂,...... ou Pₙ, après dépôt de la composition cosmétique sur un substrat en matière kératinique humaine et élimination subséquente du solvant commun (S_{c}), et (ii) une deuxième composition cosmétique comprenant au moins un solvant sélectif (Sₛ), apte à dissoudre au plus n-1 des polymères (P₁, P₂,......Pₙ) déposés sur le substrat en matière kératinique humaine et non solubilisant du substrat.

Encore un autre objet de l'invention concerne un substrat en matière kératinique humaine ou en substitut de matière kératinique humaine, revêtu d'au moins une matrice polymérique physiologiquement acceptable comportant des reliefs creux ou des excroissances, chaque matrice étant constituée d'un seul polymère.

L'invention a aussi pour objet l'utilisation d'une composition cosmétique comprenant, dans un milieu physiologiquement acceptable, n polymères (P₁, P₂,......Pₙ) avec n≥2, solubilisés dans un solvant commun (S_{c}), pour former des domaines distincts, constitués, chacun, d'un seul polymère, choisi parmi P₁, P₂,...... ou Pₙ, après dépôt de la composition cosmétique sur un substrat en matière kératinique humaine et élimination subséquente du solvant commun (S_{c}).

L'invention concerne encore un procédé cosmétique comprenant l'application, sur un substrat en matière kératinique humaine, en particulier sur des cheveux:
(a) d'une première composition cosmétique (i) comprenant, dans un milieu physiologiquement acceptable, n polymères (P₁, P₂,......Pₙ) avec 10≥n≥2, solubilisés dans un solvant commun (S_{c}), les polymères (P₁, P₂,......Pₙ) et le solvant commun (S_{c}), lequel est liquide à la température ambiante et à la pression atmosphérique, étant choisis, pour former des domaines distincts, constitués, chacun, d'un seul polymère, choisi parmi P₁, P₂,...... ou Pₙ, après dépôt de la composition cosmétique sur un substrat en matière kératinique humaine et élimination subséquente du solvant commun (S_{c}),
(b) d'une deuxième composition cosmétique (ii) comprenant au moins un solvant sélectif (Sₛ), apte à dissoudre au plus n-1 des polymères (P₁, P₂,......Pₙ) déposés sur le substrat en matière kératinique humaine et non solubilisant du substrat,
(c) éventuellement, d'une troisième composition cosmétique (iii), comprenant au moins un polymère (Pₜᵣ), avant ou après (a) ou (b) ou (d),
éventuellement encore, d'une composition cosmétique supplémentaire de déformation, de coloration, de maquillage, de démaquillage, de protection, de soin, de nettoyage ou de lavage de matière kératinique humaine, cette application étant effectuée avant ou après (a) ou (b), chacune des étapes (a), (b), (c) ou (d) étant éventuellement suivie d'un rinçage.

Avantageusement, le substrat en matière kératinique humain est formé par des cheveux.

De préférence, on met en oeuvre un nombre de polymères tel que n est inférieur ou égal à 5.

Plus préférentiellement encore, le solvant commun (S_{c}) est choisi parmi l'eau, les alcools en C₂-C₆, les alcanes diols en C₂-C₆, l'alcool benzylique, les éthers en C₂-C₆, les esters en C₂-C₆, les N-alkyl (C₁-C₄) pyrrolidones et les cétones en C₂-C₆ ainsi qu'un mélange de ces solvants.

Le solvant sélectif (Sc) peut, généralement, soit former des reliefs structurés à base de trous, soit des reliefs structurés à base de piliers.

Avantageusement, les polymères (P₁, P₂,......Pₙ) ont, chacun, une masse moléculaire moyenne en nombre allant de 1 kDa à 1000kDa; les polymères (P₁, P₂,......Pₙ) ont, chacun, une température de transition vitreuse (Tg) supérieure ou égale à 0°C.

La température de transition vitreuse correspond la température laquelle le matériau amorphe passe d'un état de solide vitreux à un état caoutchouteux. Cette température peut être mesurée par analyse thermique différentielle, dite méthode "DTA", pour Differential Thermal Analysis ou par calorimétrie différentielle, dite méthode "DSC", pour Differential Scanning Calorimetry. En particulier, la température de transition vitreuse peut être mesurée par calorimétrie différentielle "DSC" selon la norme ASTM D3418- 97.

Les polymères (P₁, P₂,......Pₙ) sont, avantageusement, choisis parmi le polystyrène, l'acide polystyrène sulfonique, les polyalkylméthacrylates en C₁-C₁₀, les polyalkylacrylates en C₁-C₁₀, les alkylamines en C₁-C₁₀, les polyalkylèneamines, les polyvinylamines, les polyvinylpyridines, les polylysines, les hydroxyalkylcelluloses en C₁-C₁₀, les hydroxyalkylguars en C₁-C₁₀, les chlorures d'hydroxyalkyl triammonium guar en C₁-C₁₀, les hydroxyalkylcelluloses triméthyl ammonium en C₁-C₁₀, les polyalkylènes oxydes en C₁-C₁₀, les polysaccharides, les acides polyacryliques, les polyacrylamides, les poly(méth-)acrylamides, les alcools polyvinyliques, les acétates de polyvinyle, les polyvinylpyrrolidones, les polyalkylèneimines en C₁-C₁₀, les polyacrylamidoalkylsulfoniques en C₁-C₁₀, les polyuréthanes, les polyesters et les acides polyphosphoriques.

La quantité relative en poids de chaque polymère (P₁, P₂,......Pₙ) dans la composition cosmétique va, avantageusement, de 0,01 à 50 %, de préférence de 0,1 à 25 %, et plus préférentiellement encore de 0,2 à 10 %.

Avantageusement, la composition selon l'invention comprend n polymères (P₁, P₂,......Pₙ) et le rapport en poids de chacun des polymères par rapport au poids total de la composition cosmétique est compris entre 0,01 et 100, de préférence entre 0,02 et 50, et plus préférentiellement encore entre 0,05 et 20.

La composition selon l'invention comprend, avantageusement, deux polymères (P₁, P₂) et le rapport en poids entre les deux polymères est compris entre 0,01 et 100, de préférence entre 0,02 et 50, et plus préférentiellement encore entre 0,05 et 20.

Les polymères (P₁, P₂,......Pₙ) comprennent, avantageusement, les couples polystyrène/ polyalkylméthacrylates en C₁-C₁₀, hydroxyalkylcelluloses triméthyl ammonium en C₁-C₁₀ / polyalkylèneimines en C₁-C₁₀ et hydroxyalkylcelluloses en C₁-C₁₀ / hydroxyalkylcelluloses triméthyl ammonium en C₁-C₁₀.

Plusieurs méthodes peuvent être envisagées pour la réalisation des compositions utilisées selon l'invention, par exemple des méthodes optiques de structuration laser, mécaniques de presse sur une matrice de polymères et physico-chimiques, comme la métallisation multicouches ou la démixtion de polymères. Selon un mode de réalisation préférentiel de l'invention, on utilise la démixtion de polymères.

Le kit selon l'invention comprend, avantageusement, en outre, (iii) une troisième composition cosmétique comprenant au moins un polymère (Pₜᵣ). Pₜᵣ est un polymère de prétraitement des fibres kératiniques, pouvant servir, par exemple, à améliorer l'adhésion des compositions utilisées dans le procédé selon l'invention ou à rendre la surface des fibres kératiniques plus lisses ou plus accrochantes.

Ce polymère sert à "unifier" la surface de la fibre et donc à contourner la variabilité naturelle de l'état de surface des cheveux pour réaliser plus facilement les structures. Il peut être n'importe quel polymère, à condition d'être insoluble dans le solvant commun Sc et également insoluble dans le solvant sélectif Ss et à condition d'être chimiquement inerte vis à vis des polymères (P1...Pn) apliqués ensuite. Par chimiquement inerte, on entend qu'aucune liaison covalente en sera créée ou détruite entre les polymères. Par exemple, on peut citer, comme polymère de prétraitement, les polyamines comme les polyalkyleneimine, les polyallylamines, les polyaminoacides, les polysaccharides, les polyacrylates, de préférence la polyethyleneimine, la polylysine.

De préférence, le kit selon l'invention, comprend, en outre, une composition supplémentaire de déformation, de coloration, de maquillage, de démaquillage, de protection, de nettoyage ou de lavage de substrat en matière kératinique humaine.

Dans la composition (i) du kit, le solvant commun (S_{c}) est choisi, avantageusement, parmi l'eau, les alcools en C₂-C₆, les alcanes diols en C₂-C₆, l'alcool benzylique, les éthers en C₂-C₆, les esters en C₂-C₆, les N-alkyl (C₁-C₄) pyrrolidones et les cétones en C₂-C₆ ainsi qu'un mélange de ces solvants.

Préférentiellement, dans la composition (i) du kit, les polymères (P₁, P₂,......Pₙ) ont, chacun, une masse moléculaire moyenne en nombre allant de 1 kDa à 1000kDa; avantageusement, les polymères (P₁, P₂,......Pₙ) sont choisis parmi le polystyrène, l'acide polystyrène sulfonique, les polyalkylméthacrylates en C₁-C₁₀, les polyalkylacrylates en C₁-C₁₀, les alkylamines en C₁-C₁₀, les polyalkylèneamines, les polyvinylamines, les polyvinylpyridines, les polylysines, les hydroxyalkylcelluloses en C₁-C₁₀, les hydroxyalkylguars en C₁-C₁₀, les chlorures d'hydroxyalkyl triammonium guar en C₁-C₁₀, les hydroxyalkylcelluloses triméthyl ammonium en C₁-C₁₀, les polyalkylènes oxydes en C₁-C₁₀, les polysaccharides, les acides polyacryliques, les polyacrylamides, les poly(méth-)acrylamides, les alcools polyvinyliques, les acétates de polyvinyle, les polyvinylpyrrolidones, les polyalkylèneimines en C₁-C₁₀, les polyacrylamidoalkylsulfoniques en C₁-C₁₀, les polyuréthanes, les polyesters et les acides polyphosphoriques.

Avantageusement, dans le kit, la quantité relative en poids de chaque polymère (P₁, P₂,......Pₙ) dans la composition cosmétique (i) va de 0,01 à 50 %, de préférence de 0,1 à 25 %, et plus préférentiellement encore de 0,2 à 10 %.

Selon un mode de réalisation avantageux du kit, la première composition (i) comprend n polymères (P₁, P₂,......Pₙ) et le rapport en poids de chacun des polymères par rapport au poids total de la composition cosmétique est compris entre 0,01 et 100, de préférence entre 0,02 et 50, et plus préférentiellement encore entre 0,05 et 20.

Selon un mode de réalisation encore plus avantageux du kit, les polymères (P₁, P₂,......Pₙ) comprennent les couples polystyrène/ polyalkylméthacrylates en C₁-C₁₀, hydroxyalkylcelluloses triméthyl ammonium en C₁-C₁₀ / polyalkylèneimines en C₁-C₁₀ et hydroxyalkylcelluloses en C₁-C₁₀ / hydroxyalkylcelluloses triméthyl ammonium en C₁-C₁₀.

Avantageusement, le substrat selon l'invention possède une matrice et des reliefs, qui comprennent chacun, indépendamment l'un de l'autre, au moins un polymère choisi parmi le polystyrène, l'acide polystyrène sulfonique, les polyalkylméthacrylates en C₁-C₁₀, les polyalkylacrylates en C₁-C₁₀, les alkylamines en C₁-C₁₀, les polyalkylèneamines, les polyvinylamines, les polyvinylpyridines, les polylysines, les hydroxyalkylcelluloses en C₁-C₁₀, les hydroxyalkylguars en C₁-C₁₀, les chlorures d'hydroxyalkyl triammonium guar en C₁-C₁₀, les hydroxyalkylcelluloses triméthyl ammonium en C₁-C₁₀, les polyalkylènes oxydes en C₁-C₁₀, les polysaccharides, les acides polyacryliques, les polyacrylamides, les poly(méth-)acrylamides, les alcools polyvinyliques, les acétates de polyvinyle, les polyvinylpyrrolidones, les polyalkylèneimines en C₁-C₁₀, les polyacrylamidoalkylsulfoniques en C₁-C₁₀, les polyuréthanes, les polyesters et les acides polyphosphoriques.

Avantageusement, concernant le substrat selon l'invention, il s'agit d'ongles, de cils, de cheveux ou de substituts de ceux-ci, ces derniers étant, de préférence, choisis parmi les faux ongles, perruques, mèches ou faux cils.

Sur le substrat le relief comprend, avantageusement, des motifs répartis de façon aléatoire ou ordonnée, ces motifs étant, avantageusement, des stries, des piliers ou des cavités.

L'invention concerne aussi une composition comprenant un couple de polymères P₁ et P₂ choisis parmi les couples polystyrène/polyalkylméthacrylates en C₁-C₁₀, hydroxyalkylcelluloses triméthyl ammonium en C₁-C₁₀ / polyalkylèneimines en C₁-C₁₀ et hydroxyalkylcelluloses en C₁-C₁₀ / hydroxyalkylcelluloses triméthyl ammonium en C₁-C₁₀.

Les compositions cosmétiques de l'invention peuvent être utilisées comme produits pour le maquillage ou pour le soin des fibres kératiniques, notamment des cheveux ou des cils. Pour les cheveux, on peut citer les produits cosmétiques comme les shampooings, les après shampooings à rincer ou non, les compositions pour permanente, défrisage, lissage, coloration, décoloration ou encore les compositions à appliquer avant ou après une coloration, une décoloration, une permanente ou un défrisage. On peut aussi citer les produits de coiffage, les laques et tous les produits de mise en forme non permanente ou de conditionnement des cheveux. Pour les cils, on peut citer les mascaras ainsi que les produits servant à allonger les cils.

Les compositions cosmétiques de l'invention peuvent être utilisées comme produits pour le maquillage et/ou le soin de la peau, des lèvres ou des ongles. On peut citer des produits comme des crèmes pour le corps, des fonds de teint, des crèmes teintées pour le visage ou des rouges à lèvres. Les produits de soin peuvent être des produits rincés ou non rincés pour le nettoyage de la peau, du visage et/ou du corps. Il peut également s'agir de produit de base pour le maquillage ou le soin, comme des produits de soin hydratant s'appliquant avant le produit de maquillage lui-même ou des produits facilitant l'étalement ou l'adhésion du maquillage.

La composition cosmétique selon l'invention peut contenir, en outre, au moins un adjuvant choisi parmi les silicones sous forme soluble, dispersée, micro-dispersée, les agents tensio-actifs non-ioniques, anioniques, cationiques et amphotères, les céramides, les glycocéramides et pseudo-céramides, les vitamines et pro-vitamines dont le panthénol, les huiles végétales, animales, minérales et synthétiques, les cires autres que les céramides, les glycocéramides et pseudo-céramides, les filtres solaires hydrosolubles et liposolubles, siliconés ou non siliconés, les pigments minéraux et organiques, colorés ou non colorés, les colorants solubles dans le milieu cosmétiquement acceptable, les agents nacrants et opacifiants, les agents séquestrants, les agents plastifiants, les agents solubilisants, les agents acidifiants, des agents alcalinisants, les agents épaississants minéraux et organiques, les agents anti-oxydants, les hydroxyacides, les agents de pénétration, les parfums et les agents conservateurs.

Bien entendu, l'homme du métier veillera choisir ce ou ces composés complémentaires, et/ ou leur quantité, de manière telle que les propriétés avantageuses de la composition selon l'invention ne soient pas ou substantiellement pas, altérées par l'adjonction envisagée.

Chaque composition selon l'invention peut être préparée par l'homme du métier sur la base de ses connaissances générales et selon l'état de la technique.

Les compositions des kits selon l'invention sont conditionnées dans des compartiments ou récipients ou dispositifs distincts, accompagnés, éventuellement, de moyens d'application appropriés, identiques ou différents, tels que les pinceaux, les brosses, les éponges.

L'invention est illustrée plus en détail dans les exemples suivants.

### EXEMPLES:

Le polystyrène et le polymethacrylate de méthyle ont un poids moléculaire de 100000.

### exemple 1

| Composition | |
|---|---|
| polystyrène | 2g |
| polymethacrylate de methyle | 1 g |
| toluene | qsp 100g |

Le solvant commun Sc est ici le toluène.

La composition est appliquée sur mèches, puis séchée.

Les mèches sont ensuite traitées avec un solvant sélectif, Ss, ici le cyclohexane, qui peut dissoudre seulement le polystyrène.

Une observation en AFM révèle que les cheveux sont couverts de structures de taille nanométrique (piliers).

### exemple 2

| Composition | |
|---|---|
| polystyrène | 2g |
| polymethacrylate de methyle | 1 g |
| toluène | qsp 100g |

Le solvant commun Sc est ici le toluène.

La composition est appliquée sur mèches, puis séchée.

Les mèches sont ensuite traitées avec un solvant sélectif, Ss, ici l'acide acétique, qui peut dissoudre seulement le polymethacrylate de méthyle.

Une observation en AFM révèle que les cheveux sont couverts de structures de taille nanométrique (creux).

### exemple 3

| Composition 1 | |
|---|---|
| Ptr : polyethyleneimine LUPASOL P @de BASF | 10g |
| Eau | qsp 100g |

| Composition 2 | |
|---|---|
| polystyrène | 2g |
| polymethacrylate de methyle | 1g |
| toluene | qsp 100g |

Le solvant commun Sc est ici le toluène.

La composition 1 est appliquée sur mèches, puis séchée.

La composition 2 est appliquée sur mèches, puis séchée.

Les mèches sont ensuite traitées avec un solvant sélectif, Ss, ici le cyclohexane, qui peut dissoudre seulement le polystyrène.

Une observation en AFM révèle que les cheveux sont couverts de structures de taille nanométrique (piliers).

## Revendications

1. Composition cosmétique comprenant, dans un milieu physiologiquement acceptable, n polymères (P₁, P₂,......Pₙ) avec 10≥n≥2, solubilisés dans un solvant commun (S_{c}), les polymères (P₁, P₂,......Pₙ) et le solvant commun (S_{c}), lequel est liquide à la température ambiante et à la pression atmosphérique, étant choisis, pour former des domaines distincts, constitués, chacun, d'un seul polymère, choisi parmi P₁, P₂,...... ou Pₙ, après dépôt de la composition cosmétique sur un substrat en matière kératinique humaine et élimination subséquente du solvant commun (S_{c}).

2. Composition selon la revendication 1, **caractérisée par le fait que** le substrat en matière kératinique humain est formé par des cheveux.

3. Composition selon l'une quelconque des revendications précédentes, **caractérisée par le fait que** n est inférieur ou égal à 5.

4. Composition selon l'une quelconque des revendications précédentes, **caractérisée par le fait que** le solvant commun (S_{c}) est choisi parmi l'eau, les alcools en C₂-C₆, les alcanes diols en C₂-C₆, l'alcool benzylique, les éthers en C₂-C₆, les esters en C₂-C₆, les N-alkyl (C₁-C₄) pyrrolidones et les cétones en C₂-C₆ ainsi qu'un mélange de ces solvants.

5. Composition selon l'une quelconque des revendications précédentes, **caractérisée par le fait que** les polymères (P₁, P₂,......Pₙ) ont, chacun, une masse moléculaire moyenne en nombre allant de 1 kDa à 1000kDa.

6. Composition selon l'une quelconque des revendications précédentes, **caractérisée par le fait que** les polymères (P₁, P₂,......Pₙ) ont, chacun, une température de transition vitreuse (Tg) supérieure ou égale à 0°C.

7. Composition selon l'une quelconque des revendications précédentes, **caractérisée par le fait que** les polymères (P₁, P₂,.....Pₙ) sont choisis parmi le polystyrène, l'acide polystyrène sulfonique, les polyalkylméthacrylates en C₁-C₁₀, les polyalkylacrylates en C₁-C₁₀, les alkylamines en C₁-C₁₀, les polyalkylèneamines, les polyvinylamines, les polyvinylpyridines, les polylysines, les hydroxyalkylcelluloses en C₁-C₁₀, les hydroxyalkylguars en C₁-C₁₀, les chlorures d'hydroxyalkyl triammonium guar en C₁-C₁₀, les hydroxyalkylcelluloses triméthyl ammonium en C₁-C₁₀, les polyalkylènes oxydes en C₁-C₁₀, les polysaccharides, les acides polyacryliques, les polyacrylamides, les poly(méth-)acrylamides, les alcools polyvinyliques, les acétates de polyvinyle, les polyvinylpyrrolidones, les polyalkylèneimines en C₁-C₁₀, les polyacrylamidoalkylsulfoniques en C₁-C₁₀, les polyuréthanes, les polyesters et les acides polyphosphoriques.

8. Composition selon l'une quelconque des revendications précédentes, **caractérisée par le fait que** la quantité relative en poids de chaque polymère (P₁, P₂,......Pₙ) dans la composition cosmétique va de 0,01 à 50 %, de préférence de 0,1 à 25 %, et plus préférentiellement encore de 0,2 à 10 %.

9. Composition selon l'une quelconque des revendications précédentes, **caractérisée par le fait qu'**elle comprend n polymères (P₁, P₂,......Pₙ) et le rapport en poids de chacun des polymères par rapport au poids total de la composition cosmétique est compris entre 0,01 et 100, de préférence entre 0,02 et 50, et plus préférentiellement encore entre 0,05 et 20.

10. Composition selon l'une quelconque des revendications précédentes, **caractérisée par le fait qu'**elle comprend deux polymères (P₁, P₂) et que le rapport en poids entre les deux polymères est compris entre 0,01 et 100, de préférence entre 0,02 et 50, et plus préférentiellement encore entre 0,05 et 20.

11. Composition selon la revendication 7, **caractérisée par le fait que** les polymères (P₁, P₂,......Pₙ) comprennent les couples polystyrène/polyalkylméthacrylates en C₁-C₁₀, hydroxyalkylcelluloses triméthyl ammonium en C₁-C₁₀ / polyalkylèneimines en C₁-C₁₀ et hydroxyalkylcelluloses en C₁-C₁₀ / hydroxyalkylcelluloses triméthyl ammonium en C₁-C₁₀.

12. Kit comprenant (i) une première composition cosmétique comprenant, dans un milieu physiologiquement acceptable, n polymères (P₁, P₂,......Pₙ) avec 10≥n≥2, solubilisés dans un solvant commun (S_{c}), les polymères (P₁, P₂,......Pₙ) et le solvant commun (S_{c}), lequel est liquide à la température ambiante et à la pression atmosphérique, étant choisis, pour former des domaines distincts, constitués, chacun, d'un seul polymère, choisi parmi P₁, P₂,...... ou Pₙ, après dépôt de la composition cosmétique sur un substrat en matière kératinique humaine et élimination subséquente du solvant commun (S_{c}), et (ii) une deuxième composition cosmétique comprenant au moins un solvant sélectif (S_{c}), apte à dissoudre au plus n-1 des polymères (P₁, P₂,......Pₙ) déposés sur le substrat en matière kératinique humaine et non solubilisant du substrat.

13. Kit selon la revendication 12 comprenant, en outre, (iii) une troisième composition cosmétique comprenant au moins un polymère (Pₜᵣ).

14. Kit selon l'une quelconque des revendications 12 ou 13, comprenant, en outre, une composition supplémentaire de déformation, de coloration, de maquillage, de démaquillage, de protection, de nettoyage ou de lavage de substrat en matière kératinique humaine.

15. Kit selon l'une quelconque des revendications 12 à 14, **caractérisé par le fait que** le solvant commun (S_{c}) est choisi parmi l'eau, les alcools en C₂-C₆, les alcanes diols en C₂-C₆, l'alcool benzylique, les éthers en C₂-C₆, les esters en C₂-C₆, les N-alkyl (C₁-C₄) pyrrolidones et les cétones en C₂-C₆ ainsi qu'un mélange de ces solvants.

16. Kit selon l'une quelconque des revendications précédentes 12 à 15, **caractérisé par le fait que** les polymères (P₁, P₂,......Pₙ) ont, chacun, une masse moléculaire moyenne en nombre allant de 1 kDa à 1000kDa.

17. Kit selon l'une quelconque des revendications précédentes 12 à 16, **caractérisé par le fait que** les polymères (P₁, P₂,......Pₙ) sont choisis parmi le polystyrène, l'acide polystyrène sulfonique, les polyalkylméthacrylates en C₁-C₁₀, les polyalkylacrylates en C₁-C₁₀, les alkylamines en C₁-C₁₀, les polyalkylèneamines, les polyvinylamines, les polyvinylpyridines, les polylysines, les hydroxyalkylcelluloses en C₁-C₁₀, les hydroxyalkylguars en C₁-C₁₀, les chlorures d'hydroxyalkyl triammonium guar en C₁-C₁₀, les hydroxyalkylcelluloses triméthyl ammonium en C₁-C₁₀, les polyalkylènes oxydes en C₁-C₁₀, les polysaccharides, les acides polyacryliques, les polyacrylamides, les poly(méth-)acrylamides, les alcools polyvinyliques, les acétates de polyvinyle, les polyvinylpyrrolidones, les polyalkylèneimines en C₁-C₁₀, les polyacrylamidoalkylsulfoniques en C₁-C₁₀, les polyuréthanes, les polyesters et les acides polyphosphoriques.

18. Kit selon l'une quelconque des revendications précédentes 12 à 17, **caractérisé par le fait que** la quantité relative en poids de chaque polymère (P₁, P₂,.....Pₙ) dans la composition cosmétique (i) va de 0,01 à 50 %, de préférence de 0,1 à 25 %, et plus préférentiellement encore de 0,2 à 10 %.

19. Kit selon l'une quelconque des revendications précédentes 12 à 18, **caractérisé par le fait que** la première composition (i) comprend n polymères (P₁, P₂,......Pₙ) et le rapport en poids de chacun des polymères par rapport au poids total de la composition cosmétique est compris entre 0,01 et 100, de préférence entre 0,02 et 50, et plus préférentiellement encore entre 0,05 et 20.

20. Kit selon la revendication 17, **caractérisé par le fait que** les polymères (P₁, P₂,......Pₙ) comprennent les couples polystyrène/polyalkylméthacrylates en C₁-C₁₀, hydroxyalkylcelluloses triméthyl ammonium en C₁-C₁₀ / polyalkylèneimines en C₁-C₁₀ et hydroxyalkylcelluloses en C₁-C₁₀ / hydroxyalkylcelluloses triméthyl ammonium en C₁-C₁₀.

21. Substrat en matière kératinique humaine ou en substitut de matière kératinique humaine, revêtu d'au moins une matrice polymérique physiologiquement acceptable comportant des reliefs creux ou des excroissances, chaque matrice étant constituée d'un seul polymère.

22. Substrat selon la revendication 21, **caractérisé par le fait que** la matrice et les reliefs comprennent chacun, indépendamment l'un de l'autre, au moins un polymère choisi parmi le polystyrène, l'acide polystyrène sulfonique, les polyalkylméthacrylates en C₁-C₁₀, les polyalkylacrylates en C₁-C₁₀, les alkylamines en C₁-C₁₀, les polyalkylèneamines, les polyvinylamines, les polyvinylpyridines, les polylysines, les hydroxyalkylcelluloses en C₁-C₁₀, les hydroxyalkylguars en C₁-C₁₀, les chlorures d'hydroxyalkyl triammonium guar en C₁-C₁₀, les hydroxyalkylcelluloses triméthyl ammonium en C₁-C₁₀, les polyalkylènes oxydes en C₁-C₁₀, les polysaccharides, les acides polyacryliques, les polyacrylamides, les poly(méth-)acrylamides, les alcools polyvinyliques, les acétates de polyvinyle, les polyvinylpyrrolidones, les polyalkylèneimines en C₁-C₁₀, les polyacrylamidoalkylsulfoniques en Ci-C₁₀, les polyuréthanes, les polyesters et les acides polyphosphoriques.

23. Substrat selon la revendication 21 ou 22, **caractérisé en ce qu'**il s'agit d'ongles, de cils, de cheveux ou de substituts de ceux-ci.

24. Substrat selon la revendication 23, **caractérisé en ce qu'**il s'agit de faux ongles, de perruques, de mèches ou de faux cils.

25. Substrat selon l'une des revendications 21 à 24, **caractérisé en ce que** le relief comprend des motifs répartis de façon aléatoire ou ordonnée.

26. Substrat selon la revendication 25, **caractérisé en ce que** les motifs sont des stries, des piliers ou des cavités.

27. Composition comprenant un couple de polymères P₁ et P₂ choisis parmi les couples polystyrène/ polyalkylméthacrylates en C₁-C₁₀, hydroxyalkylcelluloses triméthyl ammonium en C₁-C₁₀ / polyalkylèneimines en C₁-C₁₀ et hydroxyalkylcelluloses en C₁-C₁₀ / hydroxyalkylcelluloses triméthyl ammonium en C₁-C₁₀.

28. Utilisation d'une composition cosmétique comprenant, dans un milieu physiologiquement acceptable, n polymères (P₁, P₂,......Pₙ) avec n≥2, solubilisés dans un solvant commun (S_{c}), pour former des domaines distincts, constitués, chacun, d'un seul polymère, choisi parmi P₁, P₂,...... ou Pₙ, après dépôt de la composition cosmétique sur un substrat en matière kératinique humaine et élimination subséquente du solvant commun (S_{c}).

29. Procédé cosmétique comprenant l'application, sur un substrat en matière kératinique humaine, en particulier sur des cheveux:
(a) d'une première composition cosmétique (i) comprenant, dans un milieu physiologiquement acceptable, n polymères (P₁, P₂,......Pₙ) avec 10≥n≥2, solubilisés dans un solvant commun (S_{c}), les polymères (P₁, P₂,......Pₙ) et le solvant commun (S_{c}), lequel est liquide à la température ambiante et à la pression atmosphérique, étant choisis, pour former des domaines distincts, constitués, chacun, d'un seul polymère, choisi parmi Pi, P₂,...... ou Pₙ, après dépôt de la composition cosmétique sur un substrat en matière kératinique humaine et élimination subséquente du solvant commun (S_{c}),
(b) d'une deuxième composition cosmétique (ii) comprenant au moins un solvant sélectif (Sₛ), apte à dissoudre au plus n-1 des polymères (P₁, P₂,......Pₙ) déposés sur le substrat en matière kératinique humaine et non solubilisant du substrat,
(c) éventuellement, d'une troisième composition cosmétique (iii), comprenant au moins un polymère (Pₜᵣ), avant ou après (a) ou (b) ou (d),
(d) éventuellement encore, d'une composition cosmétique supplémentaire de déformation, de coloration, de maquillage, de démaquillage, de protection, de soin, de nettoyage ou de lavage de matière kératinique humaine, cette application étant effectuée avant ou après (a) ou (b), chacune des étapes (a), (b), (c) ou (d) étant éventuellement suivie d'un rinçage.
